(19) Europäisches Patentamt
European Patent Office
Office européen des brevets

(11) Veröffentlichungsnummer: **0 117 477**
**A1**

# (12) EUROPÄISCHE PATENTANMELDUNG

(21) Anmeldenummer: **84101470.7**

(22) Anmeldetag: **13.02.84**

(51) Int. Cl.³: **C 07 D 251/50**, C 07 D 251/52, C 07 D 251/70, A 01 N 43/70

(30) Priorität: **23.02.83 DE 3306197**

(43) Veröffentlichungstag der Anmeldung: **05.09.84**
**Patentblatt 84/36**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Stetter, Jörg, Dr., Gellertweg 4, D-5600 Wuppertal 1 (DE)**
Erfinder: **Gehring, Reinhold, Dr., Dasnoeckel 49, D-5600 Wuppertal 11 (DE)**
Erfinder: **Eue, Ludwig, Dr., Paul-KLee-Strasse 36, D-5090 Leverkusen 1 (DE)**
Erfinder: **Schmidt, Robert R., Dr., Im Waldwinkel 110, D-5060 Bergisch-Gladbach 2 (DE)**

## (54) Alkoximinoalkylamino-1,3,5-triazine, Verfahren zu ihrer Herstellung und ihre Verwendung als Herbizide.

(57) Neue Alkoximinoalkylamino-1,3,5-triazine der allgemeinen Formel (I)

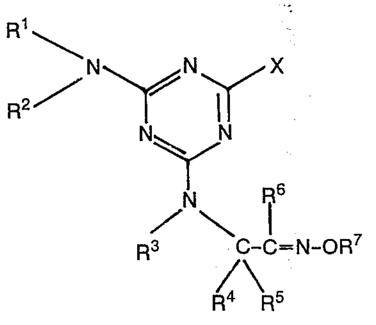

(I)

in welcher

$R^1$, $R^2$ $R^3$ unabhängig voneinander für Wasserstoff oder für gegebenenfalls substituiertes Alkyl, für Cycloalkyl oder für den Rest

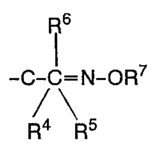

stehen,

$R^4$, $R^5$ und $R^6$ unabhängig voneinander für Wasserstoff oder für Alkyl stehen,

$R^7$ für Wasserstoff, für Alkyl, Alkenyl, Alkinyl, Cycloalkyl, Halogenalkyl oder Aralkyl steht und

X für Halogen, Cyano, Azido, Alkoxy oder Alkylthio steht, ein Verfahren zu ihrer Herstellung und ihre Verwendung als Herbizide.

BAYER AKTIENGESELLSCHAFT          5090 Leverkusen, Bayerwerk

Konzernverwaltung RP
Patentabteilung                Bi/Hed

I a

# Alkoximinoalkylamino-1,3,5-triazine, Verfahren zu ihrer Herstellung und ihre Verwendung als Herbizide

Die Erfindung betrifft neue Alkoximinoalkylamino-1,3,5-triazine, ein Verfahren zu ihrer Herstellung und ihre Verwendung als Herbizide.

Es ist bereits bekannt, daß bestimmte 1,3,5-Triazin-Derivate, wie z.B. das 2-Chlor-4-ethylamino-6-isopropylamino-1,3,5-triazin als Unkrautbekämpfungsmittel eingesetzt werden können (vgl. K.H.Büchel 'Pflanzenschutz und Schädlingsbekämpfung', G.Thieme Verlag Stuttgart 1977, S.184 f).

Die Wirkung dieser Verbindungen ist jedoch insbesondere bei niedrigen Aufwandmengen und -konzentrationen nicht immer völlig zufriedenstellend.

Es wurden nun neue Alkoximinoalkylamino-1,3,5-triazine der allgemeinen Formel (I),

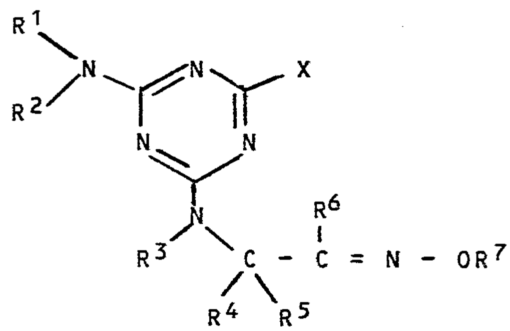

(I)

in welcher

$R^1$, $R^2$ und $R^3$ unabhängig voneinander für Wasserstoff oder für gegebenenfalls substituiertes Alkyl, für Cycloalkyl oder für den Rest

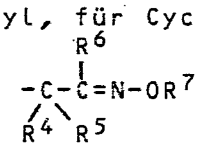

stehen,

$R^4$, $R^5$ und $R^6$ unabhängig voneinander für Wasserstoff oder für Alkyl stehen,

$R^7$ für Wasserstoff, für Alkyl, Alkenyl, Alkinyl, Cycloalkyl, Halogenalkyl oder Aralkyl steht und

X für Halogen, Cyano, Azido, Alkoxy oder Alkylthio steht,

gefunden.

Weiterhin wurde gefunden, daß man die neuen Alkoximinoalkylamino-1,3,5-triazine der allgemeinen Formel (I) erhält, wenn man 2,4-Dichlor-1,3,5-triazine der Formel (II),

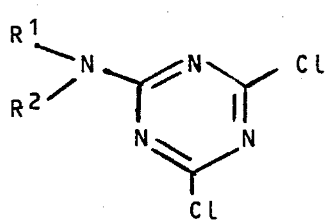

(II)

in welcher

$R^1$ und $R^2$ die oben angegebene Bedeutung haben,

gegebenenfalls in Gegenwart eines Verdünnungsmittels und gegebenenfalls in Gegenwart eines Säurebindemittels mit Alkoximinoalkylaminen der Formel (III)

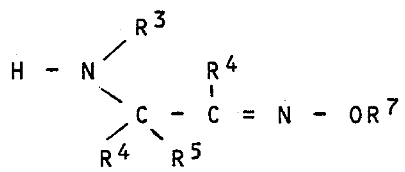

(III)

in welcher

$R^3$,$R^4$,$R^5$,$R^6$ und $R^7$ die oben angegebene Bedeutung haben,

umsetzt zu den erfindungsgemäßen 2-Chlor-1,3,5-triazinen der Formel (Ia),

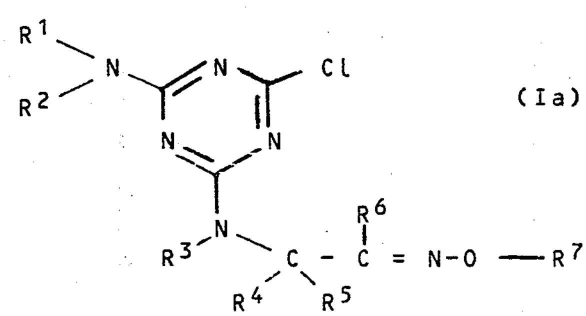

(Ia)

in welcher

$R^1, R^2, R^3, R^4, R^5$, $R^6$ und $R^7$ die oben angegebene Bedeutung haben,

und diese gegebenenfalls in einer 2.Stufe mit Verbindungen der Formel (IV)

H - X' (IV)

in welcher

X' für Halogen außer Chlor und außerdem für Cyano, Azido, Alkoxy oder Alkylthio steht,

gegebenenfalls in Gegenwart eines Verdünnungsmittels und gegebenenfalls in Gegenwart eines Säurebindemittels oder alternativ mit den entsprechenden Alkalisalzen der Formel (V),

$Me^{\oplus}$ $X'^{\ominus}$ (V)

in welcher

$X'^{\ominus}$ für das Aninon eines der unter X' beschriebenen oder angegebenen Reste steht und

$Me^{\oplus}$ für ein Alkalimetallkation steht,

gegebenenfalls in Gegenwart eines Verdünnungsmittels umsetzt zu den erfindungsgemäßen Verbindungen der Formel (Ib),

0117477

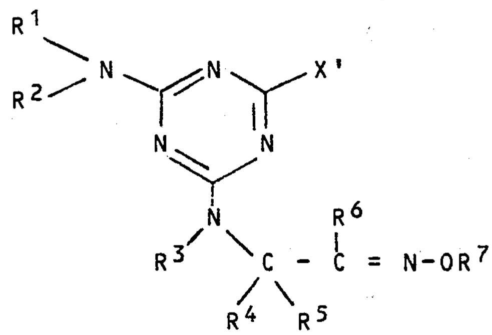

(Ib)

in welcher

$R^1, R^2, R^3, R^4, R^5, R^6, R^7$ und X' die oben angegebene Bedeutung haben.

Schließlich wurde gefunden, daß die neuen Alkoximinoalkylamino-1,3,5-triazine herbizide Eigenschaften besitzen.

Ueberraschenderweise zeigen die erfindungsgemäßen Alkoximinoalkylamino-1,3,5-triazine eine erheblich höhere herbizide Wirksamkeit sowie eine erheblich bessere Selektivität in ihrer Wirkung gegenüber Nutzpflanzen als die aus dem Stand der Technik bekannte Verbindung 2-Chlor-4-ethylamino-6-isopropylamino-1,3,5-triazin, welches eine chemisch und wirkungsmäßig naheliegende Verbindung ist (vgl. K.H. Büchel 'Pflanzenschutz und Schädlingsbekämpfung' G.Thieme Verlag Stuttgart 1977, S.184f).

Die erfindungsgemäßen Alkoximinoalkylamino-1,3,5-triazine sind durch die Formel (I) allgemein definiert. Bevorzugt sind Verbindungen der Formel (I), bei denen

$R^1, R^2$ und $R^3$ unabhängig voneinander für Wasserstoff, für geradkettiges oder verzweigtes Alkyl mit bis zu 6 Kohlenstoffatomen, für Cycloalkyl mit 3 bis 8 Kohlenstoffatomen, für geradkettiges

oder verzweigtes Cyanalkyl mit bis zu 6 Kohlenstoffatomen, für geradkettiges oder verzweigtes Halogenalkyl mit bis zu 4 Kohlenstoffatomen und bis zu 8 gleichen oder verschiedenen Halogenatomen, insbesondere Fluor und Chlor, für geradkettiges oder verzweigtes Alkoxyalkyl mit bis zu 4 Kohlenstoffatomen im Alkoxyteil und bis zu 4 Kohlenstoffatomen im Alkylteil oder für den Rest 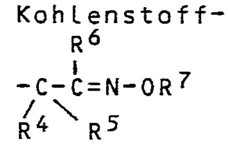 stehen, wobei

$R^4$, $R^5$ und $R^6$ unabhängig voneinander für Wasserstoff oder für geradkettiges oder verzweigtes Alkyl mit bis zu 4 Kohlenstoffatomen stehen,

$R^7$ für Wasserstoff, für geradkettiges oder verzweigtes Alkyl, Alkenyl oder Alkinyl mit jeweils bis zu 6 Kohlenstoffatomen, für geradkettiges oder verzweigtes Halogenalkyl mit bis zu 4 Kohlenstoffatomen und bis zu 8 gleichen oder verschiedenen Halogenatomen, insbesondere Fluor oder Chlor, für Cycloalkyl mit 3 bis 8 Kohlenstoffatomen oder für Aralkyl mit 6 bis 10 Kohlenstoffatomen im Arylteil und bis zu 2 Kohlenstoffatomen im Alkylteil steht und

X für Fluor, Chlor, Brom, Cyano, Azido oder für geradkettiges oder verzweigtes Alkoxy oder Alkylthio mit jeweils bis zu 4 Kohlenstoffatomen steht.

Ganz besonders bevorzugt sind Verbindungen der Formel (I), bei denen

$R^1$,$R^2$ und $R^3$ unabhängig voneinander für Wasserstoff, Methyl, Ethyl, n-Propyl, Isopropyl,n-Butyl,Isobutyl,s-Butyl,t-Butyl, Cyclopropyl, Cyclopentyl, Cyclohexyl, Cyanmethyl, 1-Cyanethyl, 2-Cyanethyl, 2-Cyan-1-propyl, 2-Cyan-2-propyl, 2-Cyan-2-butyl, 1,1,1-Trifluor-2-propyl, 2,2,2-Trifluorethyl, 2,2,2-Trichlorethyl, 3,3,3-Trifluorpropyl, 1,1,1,3,3,3-Hexafluor-2-propyl, Methoxymethyl, Ethoxymethyl, Isopropoxymethyl, Methoxyethyl, Ethoxyethyl, Isopropoxyethyl, 1-Methoxy-2-propyl, 2-Methoxy-1-propyl, 3-Methoxy-1-propyl oder für den Rest

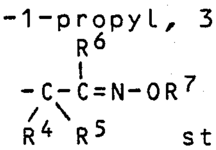

stehen, wobei

$R^4$,$R^5$ und $R^6$ unabhängig voneinander für Wasserstoff, Methyl, Ethyl, n-Propyl oder Isopropyl stehen,

$R^7$ für Wasserstoff, Methyl, Ethyl, n-Propyl, Isopropyl, n-Butyl, Isobutyl, sec.-Butyl, t-Butyl, Allyl, Propargyl, 2-Butenyl, Methallyl, Cyclopropyl, Cyclopentyl, Cyclohexyl, Cycloheptyl, 1,1,1-Trifluor-2-propyl, 2,2,2-Trifluorethyl, 2,2,2-Trichlorethyl, 3,3,3-Trifluor-1-propyl, 1,1,1,3,3,3-Hexafluor-2-propyl, Benzyl oder Phenylethyl steht und

X für Fluor, Chlor, Cyano, Azido, Methoxy, Ethoxy, Methylthio oder Ethylthio steht.

Im einzelnen seien außer den bei den Herstellungsbeispielen genannten Verbindungen die folgenden Verbindungen der allgemeinen Formel (I) genannt:

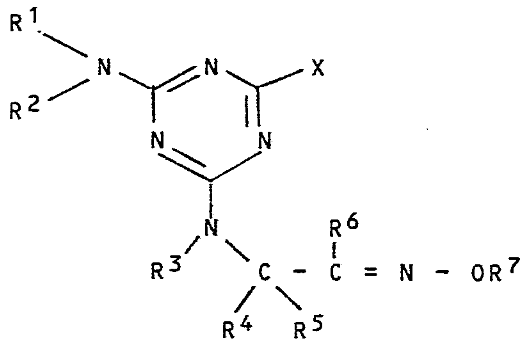

(I)

| 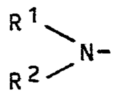 | 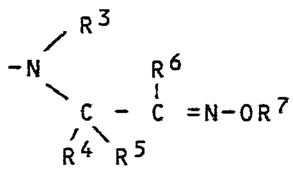 | X |
|---|---|---|
| $C_2H_5NH-$ | $-HN-CH_2-CH=NOCH_3$ | Cl |
| $C_2H_5NH-$ | 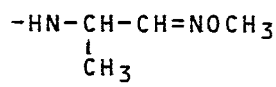 | Cl |
| $C_2H_5NH-$ | 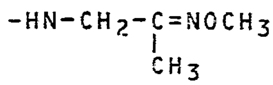 | Cl |
| $C_2H_5NH-$ | 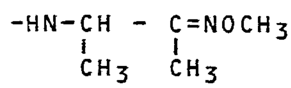 | Cl |
| $C_2H_5NH-$ | 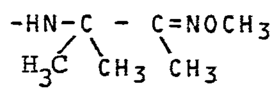 | Cl |
| $C_2H_5NH-$ | 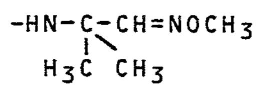 | Cl |
| $CH_3NH-$ | $-HN-CH_2-CH=NOCH_3$ | Cl |
| $CH_3NH-$ | 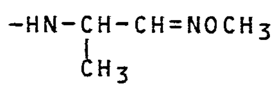 | Cl |
| $CH_3NH-$ | 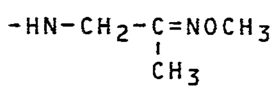 | Cl |
| $CH_3NH-$ | 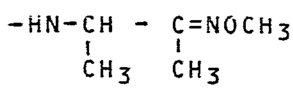 | Cl |

| 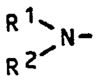 | 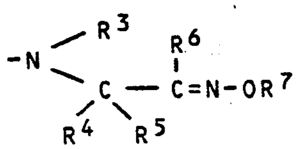 | X |
|---|---|---|
| $CH_3NH-$ | 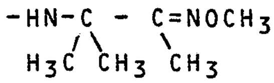 | Cl |
| $CH_3NH-$ | 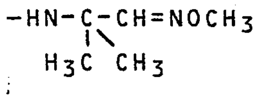 | Cl |
| $(CH_3)_2CH-NH-$ | $-HN-CH_2-CH=NOCH_3$ | Cl |
| $(CH_3)_2CH-NH-$ | 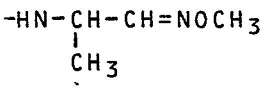 | Cl |
| $(CH_3)_3CH-NH-$ | 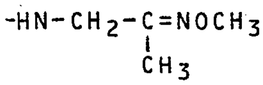 | Cl |
| $(CH_3)_2CH-NH-$ | 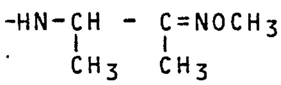 | Cl |
| $(CH_3)_2CH-NH-$ | 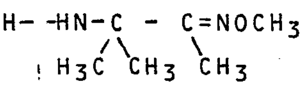 | Cl |
| $(CH_3)_2CH-NH-$ | 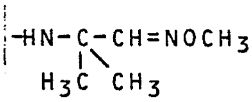 | Cl |
| $C_2H_5NH-$ | $-HN-CH_2-CH=NOC_2H_5$ | Cl |
| $C_2H_5NH-$ | 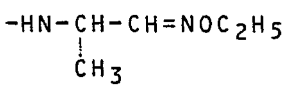 | Cl |
| $C_2H_5NH-$ | 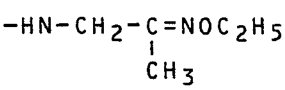 | Cl |
| $C_2H_5NH-$ | 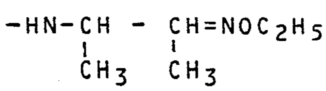 | Ol |
| $C_2H_5NH-$ | 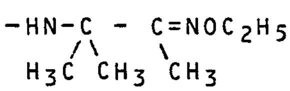 | Cl |
| $C_2H_5NH-$ | 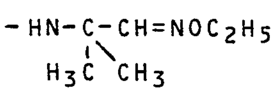 | Cl |
| $CH_3NH-$ | $-HN-CH_2-CH=NOC_2H_5$ | Cl |
| $CH_3NH-$ | 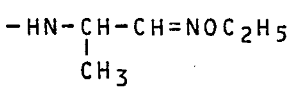 | Cl |

| 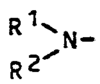 | 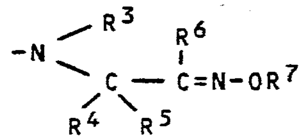 | X |
|---|---|---|
| $CH_3NH-$ | $-HN-CH_2-C=NOC_2H_5$<br>$CH_3$ | Cl |
| $CH_3NH-$ | $-HN-CH\ -\ C=NOC_2H_5$<br>$CH_3\quad CH_3$ | Cl |
| $CH_3NH-$ | $-HN-C\ -\ C=NOC_2H_5$<br>$H_3C\ CH_3\ CH_3$ | Cl |
| $CH_3NH-$ | $-HN-C-CH=NOC_2H_5$<br>$H_3C\ CH_3$ | Cl |
| $(CH_3)_2CH-NH-$ | $-HN-CH_2-CH=NOC_2H_5$ | Cl |
| $(CH_3)_2CH-NH-$ | $-HN-CH-CH=NOC_2H_5$<br>$CH_3$ | Cl |
| $(CH_3)_2CH-NH-$ | $-HN-CH_2-C=NOC_2H_5$<br>$CH_3$ | Cl |
| $(CH_3)_2CH-NH-$ | $-HN-CH\ -\ C=NOC_2H_5$<br>$CH_3\quad CH_3$ | Cl |
| $(CH_3)_2CH-NH-$ | $-HN-C\ -\ C=NOC_2H_5$<br>$H_3C\ CH_3\ CH_3$ | Cl |
| $(CH_3)_2CH-NH-$ | $-HN-C-CH=NOC_2H_5$<br>$H_3C\ CH_3$ | Cl |
| $CH_3ON=CH-CH_2NH-$ | $-HN-CH_2-CH=NOCH_3$ | Cl |
| $CH_3ON=CH-CH-NH-$<br>$CH_3$ | $-HN-CH-CH=NOCH_3$<br>$CH_3$ | Cl |
| $CH_3ON=C-CH-NH-$<br>$H_3C\ CH_3$ | $-HN-CH\ -\ C=NOCH_3$<br>$CH_3\quad CH_3$ | Cl |
| $CH_3ON=C\ -C-NH-$<br>$H_3C\ CH_3\ CH_3$ | $-HN-C\ -\ C=NOCH_3$<br>$H_3C\ CH_3\ CH_3$ | Cl |
| $CH_3ON=CH-C-NH-$<br>$H_3C\ CH_3$ | $-HN-C-CH=NOCH_3$<br>$H_3C\ CH_3$ | Cl |
| $CH_3ON=C-CH_2NH-$<br>$CH_3$ | $-HN-CH_2-C=NOCH_3$<br>$CH_3$ | Cl |

| 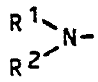 | 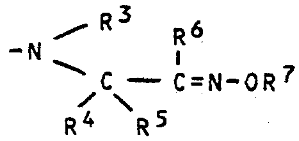 | X |
|---|---|---|
| $C_2H_5ON=CH-CH_2NH-$ | $-HN-CH_2-CH=NOC_2H_5$ | Cl |
| $C_2H_5ON=CH-CH-NH-$<br>$CH_3$ | $-HN-CH-CH=NOC_2H_5$<br>$CH_3$ | Cl |
| $C_2H_5ON=C-CH_2NH-$<br>$CH_3$ | $-HN-CH_2-C=NOC_2H_5$<br>$CH_3$ | Cl |
| $C_2H_5NH-$ | $-HN-CH_2-CH=NOCH_3$ | F |
| $C_2H_5NH-$ | $-HN-CH-CH=NOCH_3$<br>$CH_3$ | F |
| $CH_3NH-$ | $-HN-CH_2-CH=NOCH_3$ | F |
| $CH_3NH-$ | $-HN-CH-CH=NOCH_3$<br>$CH_3$ | F |
| $(CH_3)_2CH-NH-$ | $-HN-CH_2-CH=NOCH_3$ | F |
| $(CH_3)_2CH-NH-$ | $-HN-CH-CH=NOCH_3$<br>$CH_3$ | F |
| $CH_3ON=CH-CH_2NH-$ | $-HN-CH_2-CH=NOCH_3$ | F |
| $CH_3ON=CH-CH-NH-$<br>$CH_3$ | $-HN-CH-CH=NOCH_3$<br>$CH_3$ | F |
| $C_2H_5NH-$ | $-HN-CH_2-CH=NOCH_3$ | $OCH_3$ |
| $C_2H_5NH-$ | $-HN-CH-CH=NOCH_3$<br>$CH_3$ | $OCH_3$ |
| $CH_3NH-$ | $-HN-CH_2-CH=NOCH_3$ | $OCH_3$ |
| $CH_3NH-$ | $-HN-CH-CH=NOCH_3$<br>$CH_3$ | $OCH_3$ |
| $(CH_3)_2CH-NH-$ | $-HN-CH_2-CH=NOCH_3$ | $OCH_3$ |
| $(CH_3)_2CH-NH-$ | $-HN-CH-CH=NOCH_3$<br>$CH_3$ | $OCH_3$ |
| $CH_3ON=CH-CH_2NH-$ | $-HN-CH_2-CH=NOCH_3$ | $OCH_3$ |
| $CH_3ON=CH-CH-NH-$<br>$CH_3$ | $-HN-CH-CH=NOCH_3$<br>$CH_3$ | $OCH_3$ |

| 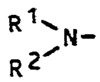 | 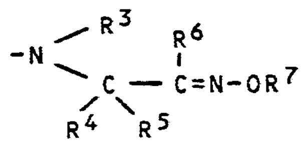 | X |
|---|---|---|
| $C_2H_5NH-$ | $-HN-CH_2-CH=NOCH_3$ | $SCH_3$ |
| $C_2H_5NH-$ | $-HN-CH-CH=NOCH_3$<br>$CH_3$ | $SCH_3$ |
| $CH_3NH-$ | $-HN-CH_2-CH=NOCH_3$ | $SCH_3$ |
| $(CH_3)_2CH-NH-$ | $-HN-CH_2-CH=NOCH_3$ | $SCH_3$ |
| $(CH_3)_2CH-NH-$ | $-HN-CH-CH=NOCH_3$<br>$CH_3$ | $SCH_3$ |
| $CH_3ON=CH-CH_2NH-$ | $-HN-CH_2-CH=NOCH_3$ | $SCH_3$ |
| $CH_3ON=CH-CH-NH-$<br>$CH_3$ | $-HN-CH-CH=NOCH_3$<br>$CH_3$ | $SCH_3$ |
| $(CH_3)_2C-NH-$<br>$CN$ | $-HN-CH_2-CH=NOCH_3$ | Cl |
| $(CH_3)_2C-NH-$<br>$CN$ | $-HN-CH-CH=NOCH_3$<br>$CH_3$ | Cl |
| $(CH_3)_2C-NH-$<br>$CN$ | $-HN-CH_2-C=NOCH_3$<br>$CH_3$ | Cl |
| $(CH_3)_2C-NH-$<br>$CN$ | $-HN-CH - C=NOCH_3$<br>$CH_3$ $CH_3$ | Cl |
| $(CH_3)_2C-NH-$<br>$CN$ | $-HN-C - C=NOCH_3$<br>$H_3C$ $CH_3$ $CH_3$ | Cl |
| $(CH_3)_2C-NH-$<br>$CN$ | $-HN-C-CH=NOCH_3$<br>$H_3C$ $CH_3$ | Cl |
| $CH_3NH-$ | $-HN-CH-CH=NOCH_3$<br>$CH_3$ | $SCH_3$ |

Verwendet man beispielsweise 2,4-Dichlor-6-ethylamino-1,3,5-triazin und 1-Methoximino-2-propylamin als Ausgangsstoffe, so läßt sich der Reaktionsablauf des erfindungsgemäßen Verfahrens durch das folgende Formelschema darstellen:

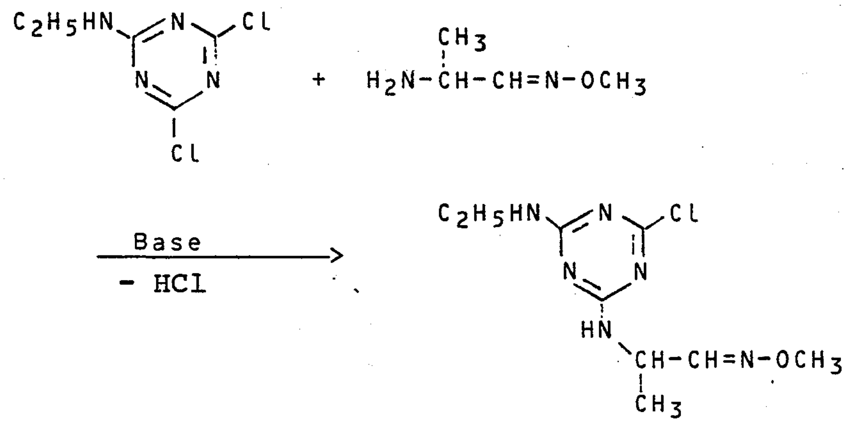


Verwendet man beispielsweise 2-Chlor-4-(1-methoximino-2-propylamino)-6-ethylamino-1,3,5-triazin und Natriummethylat als Ausgangsstoffe, so läßt sich der Reaktionsablauf des erfindungsgemäßen Verfahrens durch das folgende Formelschema darstellen (2.Stufe):

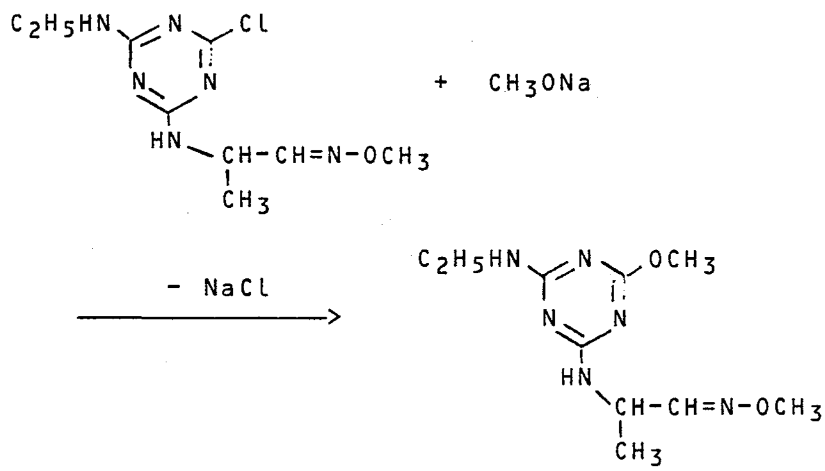

Die zur Durchführung des erfindungsgemäßen Verfahrens benötigten 2,4-Dichlor-1,3,5-triazine sind durch die Formel (II) allgemein definiert. In dieser Formel stehen $R^1$ und $R^2$ vorzugsweise für diejenigen Reste, die schon im Zusammenhang mit der Beschreibung der erfindungsgemäßen Substanzen der Formel (I) als bevorzugt genannt wurden.

Die 2,4-Dichlor-1,3,5-triazine der Formel (II) sind teilweise bekannt (vgl. z.B. R.Wegler 'Chemie derPflanzenschutz- und Schädlingsbekämpfungsmittel' Band 5, Springer Verlag, Berlin,Heidelberg,New York 1977, S.336f).

Noch nicht bekannt sind Verbindungen der Formel (II), in denen $R^1$und/oder $R^2$ für den Alkoximinoalkylrest

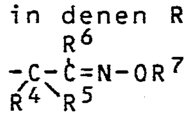

stehen, wobei $R^4$,$R^5$,$R^6$ und $R^7$ die in der Erfindungsdefinition angegebene Bedeutung haben.

Man erhält sie in prinzipiell bekannter Weise, indem man Cyanurchlorid der Formel (VI)

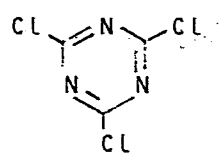

(VI)

mit Alkoximinoalkylaminen der Formel (III),

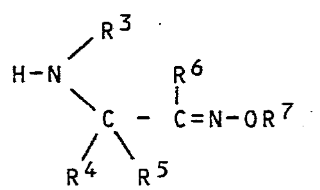

(III)

in welcher

$R^3$,$R^4$,$R^5$,$R^6$ und $R^7$ die in der Erfindungsdefinition angegebene Bedeutung haben,

gegebenenfalls in Gegenwart eines Verdünnungsmittels, wie z.B. Tetrahydrofuran und gegebenenfalls in Gegenwart eines Säurebindemittels, wie z.B. Natriumhydroxid , bei Temperaturen zwischen -20°C und +50°C umsetzt.

Die weiterhin zur Durchführung des erfindungsgemäßen Verfahrens als Ausgangsstoffe benötigten Alkoximinoalkylamine sind durch die Formel (III) allgemein definiert. In dieser Formel stehen $R^3$,$R^4$,$R^5$,$R^6$ und $R^7$ vorzugsweise für diejenigen Reste, die schon bei der Beschreibung der erfindungsgemäßen Substituenten der Formel (I) als bevorzugte Reste genannt wurden.

Die Alkoximinoalkylamine der Formel (III) sind noch nicht bekannt. Man erhält sie z.B., wenn man Aminoacetale der Formel (VII),

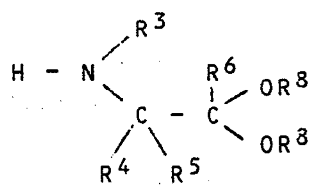

(VII)

in welcher

$R^3$,$R^4$,$R^5$ und $R^6$ die oben angegebene Bedeutung haben und

$R^8$ für geradkettiges oder verzweigtes Alkyl mit bis zu 4 Kohlenstoffatomen steht,

mit Hydroxylaminen der Formel (VIII),

$$H_2N - OR^7 \qquad (VIII)$$

in welcher

$R^7$ die oben angegebene Bedeutung hat,

oder mit deren Säureadditionssalzen der Formel (IX),

$$H_2N - OR^7 \times H^{\oplus}Y^{\ominus} \qquad (IX)$$

in welcher

$R^7$ die oben angegebene Bedeutung hat und

$Y^{\ominus}$ für das Anion einer organischen oder anorganischen Säure steht,

gegebenenfalls in Gegenwart eines Verdünnungsmittels wie z.B. Methanol und gegebenenfalls in Gegenwart eines Säurebindemittels wie z.B. Natriumacetat bei Temperaturen zwischen 20°C und 100°C umsetzt, oder indem man Alkoximinoalkylhalogenide der Formel (X),

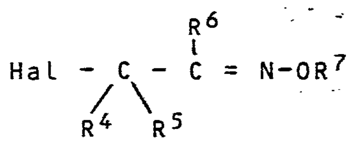

(X)

in welcher

$R^4$, $R^5$, $R^6$ und $R^7$ die oben angegebene Bedeutung haben und

Hal für Chlor oder Brom steht,

mit Aminen der Formel (XI),

$$H_2N - R^3 \qquad (XI)$$

in welcher

$R^3$ die oben angegebene Bedeutung hat,

gegebenenfalls in Gegenwart eines Verdünnungsmittels wie z.B. Wasser und gegebenenfalls in Gegenwart eines Säurebindemittels wie z.B. eines entsprechenden Ueberschusses der beteiligten Aminkomponente der Formel (XI) bei Temperaturen zwischen 0°C und 80°C umsetzt.

(Für den Fall, daß $R^3$ in der Formel (XI) für den Alkoximinoalkylrest 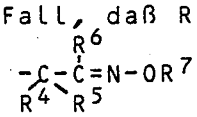 steht, sind die Amine der Formel (XI) identisch mit den Alkoximinoalkylaminen der Formel (III), wenn in diesen für $R^3$ der Wasserstoffrest steht, (=IIIa) und können nach den hier beschriebenen Verfahren erhalten werden.

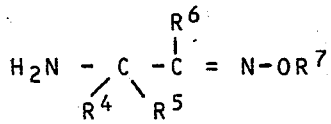 (IIIa) )

Bis-[alkoximinoalkyl]-amine der Formel (IIIb),

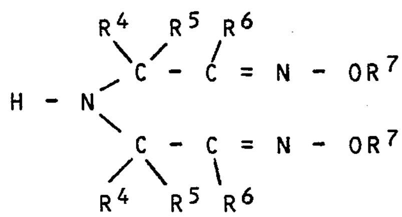 (IIIb)

in welcher

$R^4$, $R^5$, $R^6$ und $R^7$ die oben angegebene Bedeutung haben,

erhält man auch, wenn man Alkoximinoalkylhalogenide der Formel (X),

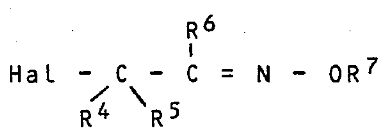 (X)

in welcher

$R^4$, $R^5$, $R^6$ und $R^7$ die oben angegebene Bedeutung haben und

Hal für Chlor oder Brom steht,

mit Ammoniak gegebenenfalls in Gegenwart eines Verdünnungsmittels wie z.B. Wasser bei Temperaturen zwischen 0°C und 80°C umsetzt.

Die Alkoximinoalkylhalogenide der Formel (X) sind bekannt (vgl. DE-OS 29 22 759 bzw. US-PS 4 337 268 [LeA 19 685]).

Die Aminoacetale bzw. -ketale der Formel (III) sind ebenfalls bekannt oder können nach prinzipiell bekannten Verfahren in einfacher Weise erhalten werden [vgl. z.B. J.Chem.Soc. 121, 1872, 1886 (1922); Liebigs Ann.Chem. 440, 139, 142 (1924)].

Die Hydroxylamine der Formel (VIII) bzw. deren Säureadditionssalze der Formel (IX), ebenso wie die Amine der Formel (XI), bei denen $R^3$ nicht für den Alkoximinoalkylrest 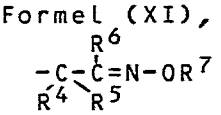 steht, sowie das Cyanurchlorid der Formel (VI) sind allgemein bekannte Verbindungen der organischen Chemie.

Die zur Durchführung der 2.Stufe des erfindungsgemäßen Verfahrens zur Herstellung der erfindungsgemäßen Verbindungen der Formel (Ib) benötigten Verbindungen bzw. deren Alkalisalze sind durch die Formeln (IV) bzw. (V) allgemein definiert. In diesen Formeln steht X' vorzugsweise für Fluor, Cyano, Azido, Methoxy, Ethoxy, Methylthio oder Ethylthio. $Me^{\oplus}$ steht vorzugsweise für ein Natrium- oder Kaliumkation.

Die Verbindungen der Formel (IV) und deren Salze der Formel (V) sind allgemein bekannte Verbindungen der organischen Chemie.

Als Verdünnungsmittel zur Durchführung des erfindungsgemäßen Verfahrens kommen vorzugsweise inerte organische Lösungsmittel infrage. Hierzu gehören insbesondere aliphatische und aromatische Kohlenwasserstoffe, wie z.B. Petrolether, Benzol oder Toluol, aliphatische oder aromatische Halogenkohlenwasserstoffe, wie z.B. Methylenchlorid, Chloroform, Tetrachlorkohlenstoff oder Chlorbenzol, Ether, wie z.B. Diethylether, Dioxan oder Tetrahydrofuran, Ketone, wie z.B. Aceton oder Butanon, Nitrile, wie z.B. Acetonitril oder Propionitril, Ester, wie z.B. Essigsäureethylester oder Amide, wie z.B. Dimethylformamid.

Das erfindungsgemäße Verfahren kann gegebenenfalls in Gegenwart eines Säurebindemittels durchgeführt werden. Als solche kommen alle üblichen anorganischen oder organischen Säurebindemittel infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine wie Triethylamin, N,N-Dimethylanilin oder Pyridin. Als Säurebindemittel kann auch ein entsprechender Ueberschuß des Alkoximinoalkylamins der Formel (III) dienen.

Die Reaktionstemperaturen können bei der Durchführung des erfindungsgemäßen Verfahrens in einem größeren Bereich variiert werden. Im allgemeinen arbeitet man bei Temperaturen zwischen 0°C und80°C, vorzugsweise bei Temperaturen zwischen 20°C und 50°C.

Bei der Durchführung des erfindungsgemäßen Verfahrens setzt man pro Mol 2,4-Dichlor-1,3,5-triazin der Formel (II) im allgemeinen 0,8 bis 1,2 Mol, vorzugsweise äquimolare Mengen des Alkoximinoalkylamins der Formel (III) und 0,8 bis 1,2 Mol, vorzugsweise äqimolare Mengen an Säurebindemittel ein.

Die Aufarbeitung und Isolierung der Verbindungen der Formel (Ia) erfolgt in üblicher Weise.

Als Verdünnungsmittel für die gegebenenfalls durchzuführende 2.Stufe des erfindungsgemäßen Verfahrens zur Herstellung der erfindungsgemäßen Verbindungen der Formel (Ib) kommen ebenfalls inerte organische Lösungsmittel infrage. Vorzugsweise verwendet man mit Wasser mischbare organische Lösungsmittel, beispielsweise Ketone, wie Aceton oder Butanon, Ether, wie Dioxan oder Tetrahydrofuran, Alkohole, wie Methanol, Ethanol oder Propanol oder Amide, wie Dimethylformamid.

Die 2.Stufe des erfindungsgemäßen Verfahrens kann gegebenenfalls in Gegenwart eines Säurebindemittels durchgeführt werden. Als solche kommen die gleichen Säurebindemittel infrage, die bei der Durchführung der 1.Stufe des erfindungsgemäßen Verfahrens aufgezählt wurden.

Die Reaktionstemperaturen können bei der 2.Stufe des erfindungsgemäßen Verfahrens ebenfalls in einem größeren Bereich variiert werden. Im allgemeinen arbeitet man zwischen 0°C und 100°C, vorzugsweise zwischen 20°C und 60°C.
Bei der Durchführung der 2.Stufe des erfindungsgemäßen Verfahrens setzt man pro Mol 2-Chlor-1,3,5-triazin der Formel (Ia) vorzugsweise 1,0 bis 1,5 Mol der Verbindung der Formel (IV) zusammen mit 1,0 bis 1,5 Mol des Säurebindemittels oder alternativ 1,0 bis 1,5 Mol eines Alkalisalzes der Formel (V), wobei die Zugabe des Säurebindemittels entfallen kann.

Die Aufarbeitung und Isolierung der Verbindungen der Formel (Ib) erfolgt in üblicher Art und Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen insbesondere eine sehr gute selektive Wirkung gegen grasartige Unkräuter, vor allem gegen Hirsearten, bei einer gleichzeitigen sehr guten Verträglichkeit gegenüber Kulturpflanzen, wie z.B. Mais oder Weizen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Im Sinne einer Verbesserung der Eigenschaften der Formulierung oder einer Wirkungsverbesserung können auch Phospholipide wie z.B. Lecithine in die Formulierung mit einbezogen werden.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H, 3H)-dion oder N-(2-Benzthiazolyl)-N,N'-dimethyl-harnstoff zur Unkrautbekämpfung in Getreide und Chloracetanilide wie z.B. Alachlor oder Metochlor zur Unkrautbekämpfung in Mais in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Für bestimmte Anwendungszwecke kann es ferner vorteilhaft sein, in die Formulierungen als weitere Zusatzstoffe pflanzenverträgliche mineralische oder vegetabilische Öle (z.B. das Handelspräparat "Sun Oil 11E") oder Ammoniumsalze wie z.B. Ammoniumsulfat oder Ammoniumrhodanid aufzunehmen.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Aussaat in den Boden eingearbeitet werden.

0117477

Die aufgewendete Wirkstoffmenge kann in größeren Bereichen schwanken. Sie hängt im wesentlichen von der Art des gewünschten Effektes ab. Im allgemeinen liegen die Aufwandmengen zwischen 0,1 und 10 kg Wirkstoff pro ha, vorzugsweise zwischen 0,25 und 5 kg/ha.

Die nachfolgenden Beispiele dienen zur weiteren Erläuterung der Erfindung.

Herstellungsbeispiele:

Beispiel 1

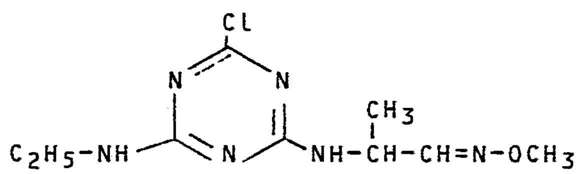

Zu einer Lösung von 38g (0,2 Mol) 2,4-Dichlor-6-ethylamino-1,3,5-triazin in 100 ml Tetrahydrofuran tropft man bei 20°C bis 25°C innerhalb von 15 Minuten 21 g (0,2 Mol) 1-Methoximino-2-propylamin. Bei exothermem Reaktionsverlauf rührt man nach beendeter Zugabe 10 Minuten unter Kühlung und tropft dann eine Lösung von 8g (0,2 Mol) Natriumhydroxid in 12 ml Wasser bei 25°C bis 35°C innerhalb von 20 Minuten zu. Nach beendeter Zugabe rührt man weitere 4 Stunden nach. Zur Aufarbeitung gießt man die Reaktionsmischung in 200 ml Wasser und extrahiert mehrfach mit Methylenchlorid. Die vereinigten organischen Extrakte werden mit Wasser gewaschen, über Natriumsulfat getrocknet und im Vakuum eingeengt. Der ölige Rückstand wird aus Ligroin kristallisiert und auf Ton getrocknet.
Man erhält 38g (74 % der Theorie) an 2-Chlor-4-(1-methoximino-2-propylamino)-6-ethylamino-1,3,5-triazin vom Schmelzpunkt 108-112°C.

Herstellung der Ausgangsverbindung:

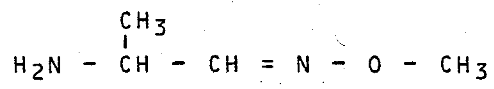 (III-1)

Zu 400 ml einer 26-prozentigen wässrigen Ammoniumhydroxid-Lösung tropft man unter Rühren 83g (0,5 Mol) 2-Brom-1-methoximinopropan und rührt nach beendeter Zugabe weitere 48 Stunden bei Raumtemperatur. Die klare Reaktionslösung wird 4 mal mit jeweils 150 ml Dichlormethan extrahiert. Die vereinigten organischen Extrakte werden über Natriumsulfat getrocknet, im Vakuum eingeengt und der Rückstand im Wasserstrahlvakuum destilliert. Man erhält 19g (37 % der Theorie) an 2-Amino-1-methoximinopropan vom Siedepunkt 45°C bei 16 mbar ( = 1-Methoximino-2-propylamin).

Beispiel 2:

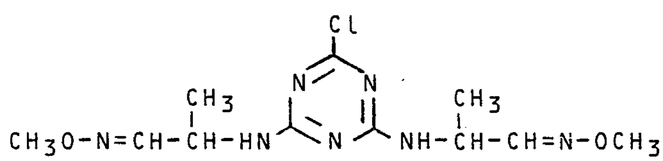

Zu einer Lösung von 18,5g (0,1 Mol) Cyanurchlorid in 100ml Tetrahydrofuran tropft man innerhalb von 20 Minuten unter Rühren und Eiskühlung bei 0°C bis 10°C 23g (0,22 Mol) 1-Methoximino-2-propylamin. Nach beendeter Zugabe rührt man weitere 3 Stunden bei Raumtemperatur. Darauf tropft man bei 25°C bis 30°C eine Lösung von 4g (0,1 Mol) Natriumhydroxid in 6 ml Wasser innerhalb von 10 Minuten zu und rührt weitere 12 Stunden bei Raumtemperatur. Zur Aufarbeitung entfernt man das Lösungsmittel im Vakuum, nimmt den Rückstand in Dichlormethan auf, wäscht mit Wasser, trocknet über Natriumsulfat und entfernt das Lösungsmittel im Vakuum. Der Rückstand wird auf einer Tonplatte abge-

0117477

preßt. Man erhält 25g (79 % der Theorie) an 2-Chlor-4,6-di-(1-methoximino-2-propylamino)-1,3,5-triazin vom Schmelzpunkt 90°C bis 92°C.

In entsprechender Weise und gemäß den allgemeinen Herstellungsangaben erhält man die folgenden Verbindungen der allgemeinen Formel (I) - s. nachfolgende Tabelle 1:

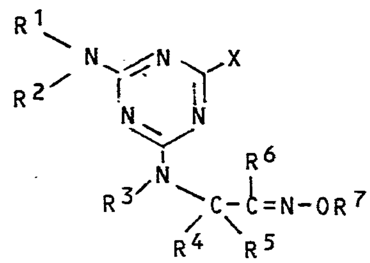

(I)

Tabelle 1

| Bsp. Nr. | $R^1$ | $R^2$ | $R^3$ | $R^4$ | $R^5$ | $R^6$ | $R^7$ | X | physikal. Eigenschaften |
|---|---|---|---|---|---|---|---|---|---|
| 3 | $C_2H_5$ | H | H | H | H | $CH_3$ | $CH_3$ | Cl | Fp:168-70°C |
| 4 | $C_2H_5$ | H | $-CH_2-\underset{CH_3}{C}=N-OCH_3$ | H | H | $CH_3$ | $CH_3$ | Cl | Fp:138-39°C |
| 5 | $C_2H_5$ | H | $-\underset{CH_3}{CH}-CH=N-OCH_3$ | $CH_3$ | H | H | $CH_3$ | Cl | $n_D^{20}=1,5444$ |
| 6 | $C_2H_5$ | H | H | $CH_3$ | H | H | $CH_3$ | $-OCH_3$ | $n_D^{20}=1,5372$ |
| 7 | $C_2H_5$ | H | H | $CH_3$ | H | H | $CH_3$ | $-SCH_3$ | $n_D^{20}=1,5797$ |
| 8 | $CH_3$ | H | H | H | H | $CH_3$ | $CH_3$ | Cl | Fp:175-78°C |
| 9 | $CH_3$ | H | H | H | H | H | $CH_3$ | Cl | Fp:185°C |
| 10 | $(CH_3)_2CH-$ | H | H | H | H | H | $CH_3$ | Cl | Fp:105-06°C |
| 11 | $(CH_3)_2CH-$ | H | H | H | H | $CH_3$ | $CH_3$ | Cl | Fp:88-90°C |
| 12 | $CH_3$ | H | H | $CH_3$ | H | H | $CH_3$ | Cl | Fp:145°C |
| 13 | $(CH_3)_2CH-$ | H | H | $CH_3$ | H | H | $CH_3$ | Cl | Fp:105-08°C |
| 14 | $CH_3O-N=CH-CH_2-$ | H | H | H | H | H | $CH_3$ | Cl | Fp:184-86°C |
| 15 | $CH_3$ | H | H | $CH_3$ | H | $CH_3$ | $CH_3$ | Cl | Fp:122-124°C |
| 16 | $(CH_3)_2CH-$ | H | H | $CH_3$ | H | $CH_3$ | $CH_3$ | Cl | Fp:129-31°C |
| 17 | $CH_3O-N=\underset{H_3C}{C}-\underset{CH_3}{CH}-$ | H | H | $CH_3$ | H | $CH_3$ | $CH_3$ | Cl | Fp:133-39°C |

Fp = Schmelzpunkt

Tabelle 1 (Fortsetzung)

| Bsp. Nr. | $R^1$ | $R^2$ | $R^3$ | $R^4$ | $R^5$ | $R^6$ | $R^7$ | X | physikal. Eigenschaften |
|---|---|---|---|---|---|---|---|---|---|
| 18 | $C_2H_5$ | H | H | H | H | H | $CH_3$ | Cl | Fp 188-192°C |
| 19 | $C_2H_5$ | H | H | $CH_3$ | H | H | $CH_3$ | $-SCH(CH_3)_2$ | Öl |
| 20 | $C_2H_5$ | H | H | $CH_3$ | H | H | $CH_3$ | $-S(CH_2)_2CH_3$ | Öl |
| 21 | $F_3C-CH_2-$ | H | H | $CH_3$ | H | H | $CH_3$ | $-SCH_3$ | Öl |
| 22 | 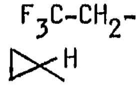 | H | H | $CH_3$ | H | H | $CH_3$ | $-SCH_3$ | Öl |
| 23 | 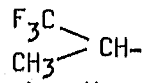 | H | H | $CH_3$ | H | H | $CH_3$ | $-SCH_3$ | Öl |
| 24 | 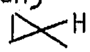 | H | H | H | H | H | $CH_3$ | $-SCH_3$ | Öl |
| 25 | 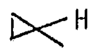 | H | H | H | H | H | $CH_3$ | Cl | Fp 135-137°C |
| 26 | 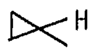 | H | H | $CH_3$ | H | H | $CH_3$ | Cl | Fp 114-116°C |
| 27 | 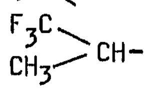 | H | H | $CH_3$ | H | H | $CH_3$ | Cl | Fp 98-100°C |
| 28 | $F_3C-CH_2-$ | H | H | $CH_3$ | H | H | $CH_3$ | Cl | Fp 144°C |
| 29 | $CH_3$ | H | H | H | H | $CH_3$ | $C_2H_5$ | Cl | Fp 181-182°C |
| 30 | $(CH_3)_2CH-$ | H | H | H | H | $CH_3$ | $C_2H_5$ | Cl | Fp 94°C |
| 31 | $C_2H_5O-N=C(CH_3)-CH_2-$ | H | H | H | H | $CH_3$ | $C_2H_5$ | Cl | Fp 161-162°C |
| 32 | $(CH_3)_2CH-$ | H | H | $CH_3$ | H | H | $C_2H_5$ | Cl | Fp 78°C |

Tabelle 1 (Fortsetzung)

| Bsp. Nr. | $R^1$ | $R^2$ | $R^3$ | $R^4$ | $R^5$ | $R^6$ | $R^7$ | X | physikal. Eigenschaften |
|---|---|---|---|---|---|---|---|---|---|
| 33 | $(CH_3)_2CH-$ | H | H | $CH_3$ | H | H | $CH_3$ | $-OCH_3$ | Öl |
| 34 | 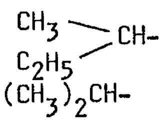 | H | H | $CH_3$ | H | H | $CH_3$ | Cl | Fp 84°C |
| 35 | $(CH_3)_2CH-$ | H | H | $CH_3$ | H | H | $CH_3$ | $-SCH_3$ | Öl |
| 36 | $(CH_3)_2CH-$ | H | H | $CH_3$ | H | H | $CH_3$ | $-N_3$ | $n_D^{20}$ 1.5450 |

Anwendungsbeispiele:

In dem nachfolgenden Anwendungsbeispiel wird die folgende Verbindung als Vergleichssubstanz herangezogen:

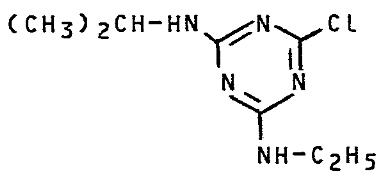

(A)

2-Chlor-4-ethylamino-6-isopropylamino-1,3,5-triazin (Atrazin)

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

In diesem Test zeigt z.B. die Verbindung gemäß Herstellungsbeispiel Nr. 1 eine deutliche Überlegenheit in der Wirksamkeit gegenüber dem Stand der Technik.

## Patentansprüche

1) Alkoximinoalkylamino-1,3,5-triazine der allgemeinen Formel (I),

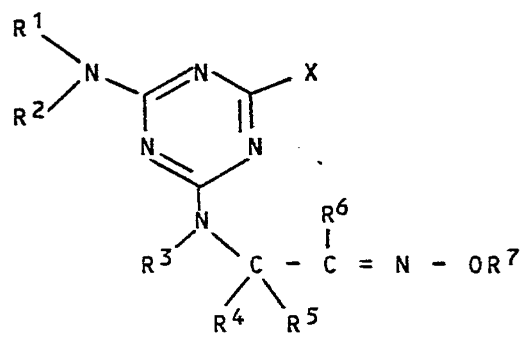

(I)

in welcher

$R^1$, $R^2$ und $R^3$ unabhängig voneinander für Wasserstoff oder für gegebenenfalls substituiertes Alkyl, für Cycloalkyl oder für den Rest 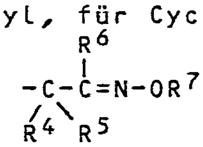 stehen,

$R^4$, $R^5$ und $R^6$ unabhängig voneinander für Wasserstoff oder für Alkyl stehen,

$R^7$ für Wasserstoff, für Alkyl, Alkenyl, Alkinyl, Cycloalkyl, Halogenalkyl oder Aralkyl steht und

X für Halogen, Cyano, Azido, Alkoxy oder Alkylthio steht.

2) Alkoximinoalkylamino-1,3,5-triazine der allgemeinen Formel (I) gemäß Anspruch 1, dadurch gekennzeichnet, daß

$R^1$,$R^2$ und $R^3$ unabhängig voneinander für Wasserstoff, für geradkettiges oder verzweigtes Alkyl mit bis zu 6 Kohlenstoffatomen, für Cycloalkyl mit 3 bis 8 Kohlenstoffatomen, für geradkettiges oder verzweigtes Cyanalkyl mit bis zu 6 Kohlenstoffatomen, für geradkettiges oder verzweigtes Halogenalkyl mit bis zu 4 Kohlenstoffatomen und bis zu 8 gleichen oder verschiedenen Halogenatomen, insbesondere Fluor und Chlor, für geradkettiges oder verzweigtes Alkoxyalkyl mit bis zu 4 Kohlenstoffatomen im Alkoxyteil und bis zu 4 Kohlenstoffatomen im Alkylteil oder für den Rest

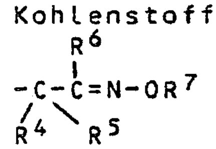

stehen, wobei

$R^4$,$R^5$ und $R^6$ unabhängig voneinander für Wasserstoff oder für geradkettiges oder verzweigtes Alkyl mit bis zu 4 Kohlenstoffatomen stehen,

$R^7$ für Wasserstoff, für geradkettiges oder verzweigtes Alkyl, Alkenyl oder Alkinyl mit jeweils bis zu 6 Kohlenstoffatomen, für geradkettiges oder verzweigtes Halogenalkyl mit bis zu 4 Kohlenstoffatomen und bis zu 8 gleichen oder verschiedenen Halogenatomen, insbesondere Fluor oder Chlor, für Cycloalkyl mit 3 bis 8 Kohlenstoffatomen oder für Aralkyl mit 6 bis 10 Kohlenstoffatomen im Arylteil und bis zu 2 Kohlenstoffatomen im Alkylteil steht und

X für Fluor, Chlor, Brom, Cyano, Azido oder für geradkettiges oder verzweigtes Alkoxy oder Alkylthio mit jeweils bis zu 4 Kohlenstoffatomen steht.

3) Alkoximinoalkylamino-1,3,5-triazine der allgemeinen Formel (I) gemäß Anspruch 1, dadurch gekennzeichnet, daß

$R^1$,$R^2$ und $R^3$ unabhängig voneinander für Wasserstoff, Methyl, Ethyl, n-Propyl, Isopropyl,n-Butyl,Isobutyl,s-Butyl,t-Butyl, Cyclopropyl, Cyclopentyl, Cyclohexyl, Cyanmethyl, 1-Cyanethyl, 2-Cyanethyl, 2-Cyan-1-propyl, 2-Cyan-2-propyl, 2-Cyan-2-butyl, 1,1,1-Trifluor-2-propyl, 2,2,2-Trifluorethyl, 2,2,2-Trichlorethyl, 3,3,3-Trifluorpropyl, 1,1,1,3,3,3-Hexafluor-2-propyl, Methoxymethyl, Ethoxymethyl, Isopropoxymethyl, Methoxyethyl, Ethoxyethyl, Isopropoxyethyl, 1-Methoxy-2-propyl, 2-Methoxy-1-propyl, 3-Methoxy-1-propyl oder für den Rest

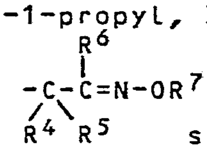

stehen, wobei

$R^4$,$R^5$ und $R^6$ unabhängig voneinander für Wasserstoff, Methyl, Ethyl, n-Propyl oder Isopropyl stehen,

$R^7$ für Wasserstoff, Methyl, Ethyl, n-Propyl, Isopropyl, n-Butyl, Isobutyl, sec.-Butyl, t-Butyl, Allyl, Propargyl, 2-Butenyl, Methallyl, Cyclopropyl, Cyclopentyl, Cyclohexyl, Cycloheptyl, 1,1,1-Trifluor-2-propyl, 2,2,2-Trifluorethyl, 2,2,2-Trichlorethyl, 3,3,3-Trifluor-1-propyl, 1,1,1,3,3,3-Hexafluor-2-propyl, Benzyl oder Phenylethyl steht und

X für Fluor, Chlor, Cyano, Azido, Methoxy, Ethoxy, Methylthio oder Ethylthio steht.

4) 2-Chlor-4-(1-methoximino-2-propylamino)-6-ethylamino-1,3,5-triazin der Formel

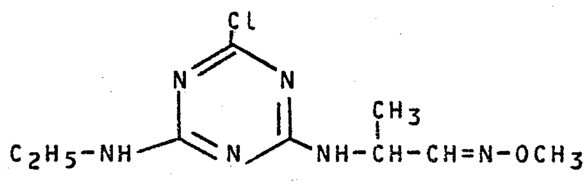

gemäß Anspruch 1.

5) Verfahren zur Herstellung von Alkoximinoalkylamino-1,3,5-triazinen der allgemeinen Formel (I),

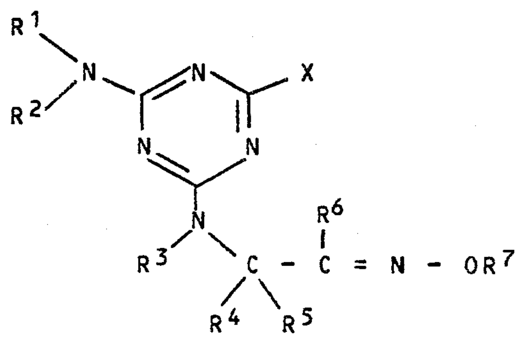

(I)

in welcher

$R^1$,$R^2$ und $R^3$ unabhängig voneinander für Wasserstoff oder für gegebenenfalls substituiertes Alkyl, für Cycloalkyl oder für den Rest 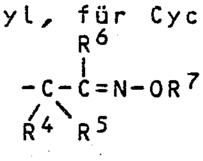 stehen,

$R^4$,$R^5$ und $R^6$ unabhängig voneinander für Wasserstoff oder für Alkyl stehen,

$R^7$ für Wasserstoff, für Alkyl, Alkenyl, Alkinyl, Cycloalkyl, Halogenalkyl oder Aralkyl steht und

X für Halogen, Cyano, Azido, Alkoxy oder Alkylthio steht,

dadurch gekennzeichnet, daß man 2,4-Dichlor-1,3,5-triazine der Formel (II)

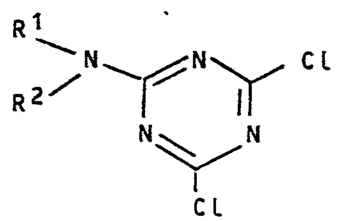

(II)

in welcher

$R^1$ und $R^2$ die oben angegebene Bedeutung haben,

gegebenenfalls in Gegenwart eines Verdünnungsmittels und gegebenenfalls in Gegenwart eines Säurebindemittels mit Alkoximinoalkylaminen der Formel (III)

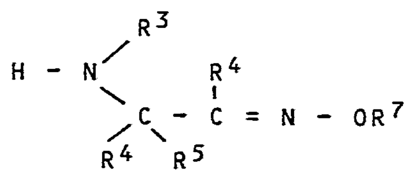

(III)

in welcher

$R^3$, $R^4$, $R^5$, $R^6$ und $R^7$ die oben angegebene Bedeutung haben,

umsetzt zu den erfindungsgemäßen 2-Chlor-1,3,5-triazinen der Formel (Ia),

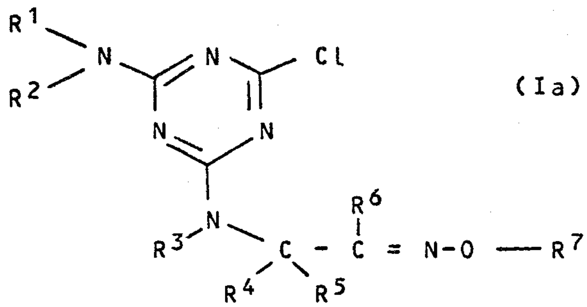

(Ia)

in welcher

$R^1$, $R^2$, $R^3$, $R^4$, $R^5$, $R^6$ und $R^7$ die oben angegebene Bedeutung haben,

und diese gegebenenfalls in einer 2. Stufe mit Verbindungen der Formel (IV)

H - X' (IV)

in welcher

X' für Halogen außer Chlor und außerdem für Cyano, Azido, Alkoxy oder Alkylthio steht,

gegebenenfalls in Gegenwart eines Verdünnungsmittels und gegebenenfalls in Gegenwart eines Säurebindemittels oder alternativ mit den entsprechenden Alkalisalzen der Formel (V),

$Me^{\oplus}$ $X'^{\ominus}$ (V)

in welcher

$X'^{\ominus}$ für das Aninon eines der unter X' beschriebenen oder angegebenen Reste steht und

$Me^{\oplus}$ für ein Alkalimetallkation steht,

gegebenenfalls in Gegenwart eines Verdünnungsmittels umsetzt zu den erfindungsgemäßen Verbindungen der Formel (Ib),

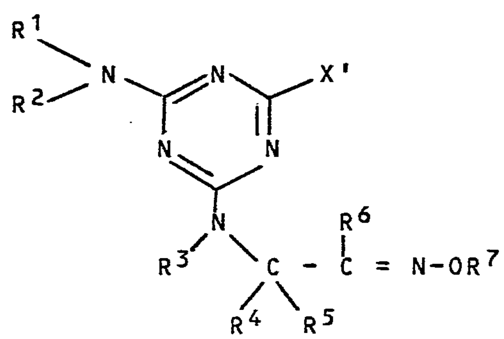

(Ib)

in welcher

$R^1$, $R^2$, $R^3$, $R^4$, $R^5$, $R^6$, $R^7$ und X' die oben angegebene Bedeutung haben.

6) Herbizide Mittel, gekennzeichnet durch einen Gehalt an mindestens einem Alkoximinoalkylamino-1,3,5-triazin der Formel (I) gemäß Anspruch 1.

7) Verwendung von Alkoximinoalkylamino-1,3,5-triazinen der Formel (I) gemäß Anspruch 1 zur Bekämpfung von Unkräutern.

8) Verfahren zur Herstellung von herbiziden Mitteln, dadurch gekennzeichnet, daß man Alkoximinoalkylamino-1,3,5-triazine der Formel (I) gemäß Anspruch 1 mit Streckmitteln und/oder oberflächenaktiven Mitteln vermischt.

Europäisches Patentamt

# EUROPÄISCHER RECHERCHENBERICHT

0117477

Nummer der Anmeldung

EP 84 10 1470

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| A | GB-A-1 068 036 (DEGUSSA)<br>* Seiten 1,2 *<br>----- | 1,6 | C 07 D 251/50<br>C 07 D 251/52<br>C 07 D 251/70<br>A 01 N 43/70 |
| | | | RECHERCHIERTE SACHGEBIETE (Int. Cl. 3) |
| | | | C 07 D 251/00 |

Der vorliegende Recherchenbericht wurde für alle Patentansprüche erstellt.

| Recherchenort | Abschlußdatum der Recherche | Prüfer |
|---|---|---|
| DEN HAAG | 23-05-1984 | VAN BIJLEN H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82